# EUROPEAN PATENT APPLICATION

(11) **EP 4 152 002 A1**
(43) Date of publication of application: **22.03.2023**
(21) Application number: 21197768.1
(22) Date of filing: 20.09.2021
(51) Int. Cl.: G01N 33/543

(54) **HETEROGENEOUS RECEPTOR-LIGAND ASSAY DEVICE, IN PARTICULAR LATERAL FLOW TESTING DEVICE, AND RELATED DEVICES AND METHODS**

(71) Applicant: ams AG, 8141 Premstätten (AT)
(72) Inventor: STOCKMEIER, Thomas, 8141 Premstätten (AT); ROSSI, Markus, 8645 Jona (CH)
(74) Representative: OSRAM GmbH - GC IP

(57) **Abstract**

The monitoring device for use in a heterogeneous receptor-ligand assay for detecting presence or amount of an analyte in a liquid comprises
- one or more electrical consumers (41; D, T), e.g., a first light source (41);
- a power supply unit (50) for supplying said one or more electrical consumers (41; D, T) with electrical energy.

The power supply unit (50) comprises an energy receiving unit (51) for receiving energy from an external device (200), such as from a mobile phone. The energy receiving unit (51) can comprise a coil for wireless power transfer by electromagnetic induction. The first light source (41) can in particular be a vertical-cavity surface-emitting laser.

The heterogeneous receptor-ligand assay device (100) comprises in addition to the monitoring device a carrier (10) held in the carrier holder (10a).

## Description

The invention relates to the detection of the presence of a target substance in a liquid sample, more particularly, it relates to heterogeneous receptor-ligand assays, such as to lateral flow tests. More specifically, it relates to methods and apparatuses according to the opening clauses of the claims. Heterogeneous receptor-ligand assays like, for example, lateral flow tests, find application in medicine and in biochemistry and other areas, e.g., in the detection of pathogens and/or viruses in a body liquid.

Heterogeneous receptor-ligand assays are widely used for detection of an analyte (the receptor) according to the lock-and-key principle, the ligand binding specifically to the analyte. In particular, immobilized biospecific ligands can be used therefor.

Heterogeneous receptor-ligand assays are also known as heterogeneous receptor-ligand binding assays or heterogeneous ligand binding assays.

Various detection principles can be applied, such as magnetometry, electrochemistry and optical principles. Optical principles are particularly important to the disclosed invention; they include, e.g., fluorimetry and colorimetry.

The ligand can function as a marker for detecting the analyte when bonded to the ligand. However, in most instances, the ligand is hard to detect. Therefore, in order to facilitate detection of the analyte (bonded to the ligand), the ligand is bonded to a marking agent, thus forming a conjugate; and in the assay, the analyte is bonded to the conjugate, more particularly to the ligand portion of the conjugate. Thus, the conjugate then functions as a marker, due to the marking agent portion of the conjugate. The marking agent can be, e.g., a luminescent agent, such as a fluorescent agent, e.g., a fluorescent molecule; or a coloring agent, such as a gold particle or a latex particle or a dye molecule. It is noted that, deviating from the herein proposed use of the term "marker", experts in the field use the term "marker" in most cases for what is herein referred to as "marking agent".

The assays can be designed in various ways, for example as lateral flow assays, as through flow assays or as assays where the ligand does not flow but remains in a cavity.

Most widely used, in particular for rapid analysis, are lateral flow assays, also referred to as lateral flow tests (LF tests, LFT). Typically, the flow of the liquid sample is due to capillary forces, which may be accomplished by, e.g., the use of felt-like or fibrous materials or microstructured materials or microchannels.

The most prominen heterogeneous receptor-ligand assays are immunoassays which rely on the specific interaction of antibodies with antigens. Further typical applications are based on hybridisation of nucleic acids or on lectine-saccaride interaction.

The assay herein described can be an assay of any of the above or below kinds. However, in the following, we shall mainly focus on the example of a lateral flow test such as on a lateral flow immunochromatographic assay.

The following example of a lateral flow test describes, how the presence of an analyte in a liquid sample can be detected, e.g., can be visually detected:
The liquid sample is applied to a carrier, such as to a strip, more particularly to a sample pad of the carrier, and runs along the carrier, across a pad referred to as conjugate pad (or conjugate release pad) providing markers (e.g., conjugates) which bond to the analyte. Then the liquid sample runs further to a test area in which analyte bonding agents are fixed to the carrier, such that at least a portion of the analyte stays there instead of continuing running across the carrier. The markers bonded to the analyte, do, when bonded to the analyte bonding agent, show a specific color (first color). In some cases, they can show this first color by themselves. In other cases, such as when fluorescent markers are used, they can show this first color in reaction to an excitation, typically by incident light, more particularly when the test area is illuminated by light of an excitation wavelength resulting in emission of light of an emission (fluorescent) wavelength which is usually different from, typically longer than, the excitation wavelength and which generally corresponds to the first color.

Detecting that first color can mean a positive test result (non-competitive assay): The analyte is contained in the liquid; in particular when a threshold color intensity of the first color is exceeded.

For control purposes, such as for validation of the test, the carrier can have a control area in which marker bonding agents are fixed to the carrier, such that markers stay there instead of continuing running across the carrier, e.g., in order to be finally taken up by a porous material, such as by a porous material comprised in a wicking pad. The markers, when bonded to the marker bonding agent, also show a specific color (second color), again, by themselves or upon excitation, as described above for the first color, and detection can be accomplished analogously. The first and second colors can be equal or different. Typically, the first and second colors are identical.

In presence of the analyte in the liquid, the test area, in case of a non-competitive assay, will show the first color after the liquid had enough time to reach the test area. And the intensity of this color can be indicative of the concentration of the analyte in the liquid sample. When the liquid sample had enough time to reach the control area, it will also show a specific color, namely the second color - which is particularly important in cases where the liquid sample does not contain any of the analyte, namely in order to ensure that the test has generally worked and that it is correct that the test area does not show the first color because the liquid lacks analyte. As indicated above, usually, the first and second colors are identical.

A test area can be, e.g., line-shaped or dot-shaped.

Furthermore, an assay can comprise more than one test areas, such as an array of dot-shaped test areas. Each test area can be dedicated to detection of a different analyte, such as by having different analyte bonding agents (specific to a respective analyte each) fixed to the carrier in the different test areas. A parallel and/or simultaneous detection of two or more analytes in the liquid is possible this way.

A merely visual detection of the color changes bears the risk of false readings and poor reproducibility. But it can be carried out on-site and without further tools and apparatuses; e.g., without dedicated apparatuses into which the carrier would be inserted for an automated reading. Such apparatuses are usually bulky and rather immobile, let alone expensive.

An automated detection of the color changes can have a good reproducibility. But this usually requires said additional apparatuses dedicated for that task which tend to be expensive, bulky and rather immobile and available in hospitals or medical centers only.

It would be desirable to enable color detection in heterogeneous receptor-ligand assays, e.g., in LF testing, in high quality, in particular having a good reproducibility, on-site, more specifically without requiring said dedicated apparatuses.

Furthermore, depending on the (first) color, it may be impossible to visually perceive the color intensity changes, such as when the color is outside the visible range, like, e.g., for wavelengths (for the first and/or second color) below about 380 nm or above about 750 nm.

It is noted that the term "color" as used herein relates to (electromagnetic) wavelengths and is not limited to the visible range. Already below 400 nm and above 700 nm, detection by vision will usually not result in satisfactory results, such that assisted or automated detection will be preferred.

Likewise, the term "light" used herein means electromagnetic waves in general. More particularly, it refers to electromagnetic waves in the range including the infrared range, the visible range and the ultraviolet range.

It has been contemplated that a single-use and/or disposable and/or portable monitoring device and heterogeneous receptor-ligand assay device, e.g., LF test device, would be desirable which can be used on-site and/or which enables high accuracy color detection. In particular, such assays / testing shall be enabled without requiring said dedicated apparatuses. More specifically, it has been contemplated that such heterogeneous receptor-ligand assays, e.g., LF tests, can make use of widely available tools, such as of mobile communication devices and/or computing devices, e.g., of mobile phones or tablet computers; or of power banks, such as power banks for use with said mobile communication devices and/or computing devices. However, the assays / tests and devices can be designed such that said widely available tools do not constitute a part of the assay or test which would have to be required to fulfill specific regulatory requirements, such as ISO 13485.

Furthermore, the devices shall be very cost-efficient, i.e. be manufacturable at very low cost. And it shall be possible to readily mass-produce the devices. Furthermore, they shall be very small. And they should have a long shelf life. Finally, they shall be insensitive to environmental influences, such as to temperature changes or high temperatures and/or to humidity changes and/or to high humidity.

Still furthermore, they should be environment-friendly.

A possible object of the invention is to create new ways of detecting color and/or color changes, in particular new ways of detecting, in particular optically detecting, substances, more particularly new heterogeneous receptor-ligand assays, such as new ways of LF testing.

Another possible object of the invention is to do so in an automated fashion.

Another possible object of the invention is to do so in a particularly cost-efficient fashion.

Another possible object of the invention is to do so at a particularly high accuracy (reproducibility).

Another possible object of the invention is to do so at a particularly high sensitivity.

Another possible object of the invention is to do so in a particularly energy-efficient way.

Another possible object of the invention is to do so using an assay device which has a long shelf life.

Another possible object of the invention is to do so without requiring dedicated apparatuses as described above.

Another possible object of the invention is to do so using portable devices only.

Another possible object of the invention is to do so using an assay device which is particularly small.

Another possible object of the invention is to do so in an environment-friendly way.

Another possible object of the invention is to do so using devices for which admission regarding regulatory requirements (such as requirements according to ISO13485) can be meaningfully obtained, in particular in the sense that said devices can be readily obtained and produced, respectively, over a long period of time, such as over many years.

Another possible object of the invention is to create ways of detecting color and/or color changes, in particular new ways of detecting, in particular optically detecting, substances, more particularly new heterogeneous receptor-ligand assays, such as new ways of LF testing, which can be carried out on-site.

Furthermore, corresponding devices shall be provided as well as corresponding methods and uses- This concerns in particular monitoring devices for use in heterogeneous receptor-ligand assays (e.g., in lateral flow testing) and/or heterogeneous receptor-ligand assay devices, such as LFT devices.

Another possible object of the invention is to provide monitoring devices and/or LFT devices which are single-use and/or disposable.

Another possible object of the invention is to provide monitoring devices and/or heterogeneous receptor-ligand assay devices which are very small and lightweight.

Another possible object of the invention is to provide monitoring devices and/or heterogeneous receptor-ligand assay devices which can be used on-site.

Another possible object of the invention is to provide monitoring devices and/or heterogeneous receptor-ligand assay devices which are manufacturable at very low cost.

Another possible object of the invention is to provide monitoring devices and/or heterogeneous receptor-ligand assay devices which can be readily mass-produced.

Another possible object of the invention is to provide monitoring devices and/or heterogeneous receptor-ligand assay devices which have a long shelf life and/or which are insensitive to environmental influences, such as to temperature changes or high temperatures and/or to humidity changes and/or to high humidity.

Another possible object of the invention is to provide monitoring devices and/or heterogeneous receptor-ligand assay devices which are disposable devices and/or which are single-use devices.

Another possible object of the invention is to provide monitoring devices and/or heterogeneous receptor-ligand assay devices which are portable.

Another possible object of the invention is to provide monitoring devices and/or heterogeneous receptor-ligand assay devices which are environment-friendly.

Another possible object of the invention is to provide monitoring devices and/or heterogeneous receptor-ligand assay devices for which admission regarding regulatory requirements (such as requirements according to ISO13485) can be meaningfully obtained, in particular in the sense that said devices can be readily obtained and produced, respectively, over a long period of time, such as over many years.

Further objects and various advantages emerge from the description and embodiments herein described.

At least one of these objects is at least partially achieved by devices and methods according to the patent claims.

The power consumption of electrical consumers of a device for automated color detection, such as in an heterogeneous receptor-ligand assay, e.g., in LFT, has been identified as a particularly important issue. And this applies in particular to the one or more light sources used in the device for the color detection.

There are two aspects to the invention which may be combined with one another showing beneficial synergies, but which can also be separate invention aspects.

The first aspect concerns the power supply of the device. It is suggested to include an energy receiving unit in the power supply. This way, the device can receive energy from an external device. And this way, electrical consumers of the device, in particular all of them, can be powered by an external device. For example, the device may be devoid of a battery which would be included in the device. This is environment-friendly. And the device can be smaller, more lightweight, and problems associated with ageing of a battery (limited shelf life, susceptibility to temperature and/or humidty) can be avoided.

For example, while carrying out an assay (conducting a measurement process), energy from the external device is received by the energy receiving unit and, simultaneously, consumed by the electrical consumers.

In particular, in the first aspect, the monitoring device for use in a heterogeneous receptor-ligand assay, e.g., for use in a lateral flow test, for detecting presence or amount of an analyte in a liquid can comprise
- one or more electrical consumers;
- a power supply unit for supplying said electrical consumers with electrical energy;
the power supply unit comprising an energy receiving unit for receiving energy from an external device.

The second aspect concerns a light source included in the device, more particularly a light source used for the color detection in the device. It is suggested that the device includes a light source which is a vertical-cavity surface-emitting laser (VCSEL). The energy consumption of a VCSEL can be extremely low, for example only 2 to 3 mW, where for a comparable performance, a light emitting diode (LED) would consume around 40 mW. One fact leading to this advantage is the strong directivity of the light emitted from a VCSEL.

In particular, in the second aspect, the monitoring device for use in a heterogeneous receptor-ligand assay, e.g., in a lateral flow test, for detecting presence or amount of an analyte in a liquid can comprise
- a housing, the housing comprising a carrier holder for holding a carrier for transport of the liquid;
- at least a first light source which is a VCSEL;
wherein the first light source is structured and arranged to illuminate with light a test range in a test area of a carrier held in the carrier holder. Accordingly, the VCSEL can be used for color detection in the heterogeneous receptor-ligand assay.

More particularly, the monitoring device can comprise a unit for color detection comprising at least said first light source. The unit for color detection can furthermore comprise a detecting unit. In some embodiments, the first light source is a constituent of the detecting unit. In other embodiments, the first light source is separate from rhte detecting unit.

The unit for color detection can be accomodated or integrated in the housing.

From a color detected by the unit for color detection, in particular from an intensity of the color, presence or amount of the analyte in the liquid can be inferred, e.g., as explained above.

The two aspects can be well combined, as the very low power consumption of the VCSEL makes possible to use widely available tools as mentioned above, e.g., mobile phones or tablet computers, and/or widespread power transmission standards, for supplying the device with power, in particular in a wireless fashion. This can mean a tremendous advantage.

In some embodiments, the external device is a mobile communication device and/or a mobile computing device, e.g., a mobile phone, such as a smart phone.

In some embodiments, the energy receiving unit is operable for receiving energy in a wireless fashion. This can simplify handling and operation.

In some embodiments, the energy receiving unit comprises a coil (in particular: induction coil) for wireless power transmission by electromagnetic induction. This way, electromagnetic fields, in particular electromagnetic waves, emitted from the external device, can be used to transmit energy into the energy receiving unit.

For example, electromagnetic waves in the radio frequency (RF) range can be used to transfer energy into the energy receiving unit. E.g., techniques used in radio frequency identification (RFID) and/or in near-field communication (NFC) can be used. Such techniques are well established and can be used in the monitoring device, provided the overall power consumption of the monitoring device is sufficiently low. And this can be accomplished in particular by using one or more VCSELs as the one or more light sources for the color detection.

In some embodiments, the power supply unit comprises an AC-DC converter (also known as rectifier). By means of the AC-DC converter, AC (alternating current) power signals produced by the energy receiving unit, e.g., from inductive power transmission, such as via the coil, e.g., from alternating electromagnetic fields, such as RF fields, can be transformed into DC (direct current) by means of which the electrical consumers are supplied by the power supply unit. The direct current can in particular have a voltage of at most 6 V, more particularly of at most 4 V, and still more particularly of at most 3 V. VCSELs can be operable at as little as 3 V or even 2 V.

In some embodiments, the energy receiving unit is operable for receiving energy in a wirebound fashion. This can simplify handling and operation, but requires the presence of a suitable wire or connector.

In some embodiments, the energy receiving unit comprises a connector, e.g., a socket or a plug. This connector can be provided for cooperating with another connector associated with the external device, e.g., a connector of the external device or a connector of a wire interconnecting the monitoring device with the external device.

Electrical power can be transferred from the external device to the energy receiving unit via the connector. A galvanic interconnection between the external device and the energy receiving unit can be established via the connector.

For the wirebound energy transmission, widely used standards can be used, e.g., a universal serial bus (USB) standard.

In some embodiments, the device is devoid of a battery. This way, the device can be designed to be particularly small. And this can be particularly environment-friendly. And it can have a long shelf life.

In some embodiments, the device is devoid of an energy storage device for storing electrical energy. This can be particularly environment-friendly.

In some embodiments, the device can be devoid of an energy storage device for storing electrical energy having a storage capacity which exceeds 200%, in particular 100%, more particularly 50%, still more particularly 20% of an amount of energy consumed by a single measurement process of the heterogeneous receptor-ligand assay. And in some - similar - embodiments, the device can be devoid of an energy storage device for storing electrical energy having a storage capacity which exceeds 20 mWs, in particular 5 mWs, more particularly 2 mWs, still more particularly 1 mWs, even more particularly 0.4 mWs. These embodiments may distinguish the herein described device over other devices having an energy storage unit, e.g., similar to a battery. And the herein described device may, in instances, comprise, e.g., as a constituent of the power supply unit (e.g., as a constituent of the AC-DC converter), a means for ripple reduction, such as for reducing AC components in direct current signals supplied to said one or more electrical consumers. This means can be, e.g., a capacitor (filter capacitor), and usually has a storage capacity which is very small, in particular smaller than the storage capacities mentioned above.

In some embodiments, the monitoring device comprises a housing, the housing comprising a carrier holder for holding a carrier for transport of the liquid, in particular for transport of the liquid by capillary forces.

In some embodiments, the electrical consumers comprise at least a first light source, such as the VCSEL (second aspect). The first light source can in particular be used in the color detection.

In some embodiments, the electrical consumers comprise at least one further light source. The one or more further light sources can in particular be used in the color detection. The one or more further light sources can in particular be VCSELs.

In some embodiments, the first light source is structured and arranged to illuminate with light a test range in a test area of a carrier held in the carrier holder.

In some embodiments, the electrical consumers comprise a detecting unit, the detecting unit comprising at least one detector, in particular a light detector, e.g., a photo diode. The detecting unit can produce a measurement signal, in particular wherein the measurement signal is associated with presence or intensity of a specific color. The measurement signal can be associated with said presence or amount of said analyte in said liquid.

In some embodiments, light emitted from the liquid, more particularly from the test range, in reaction to illumination thereof with light from the first light source can be detected by the detecting unit, e.g., the detecting unit comprising a light detector, such as a photodiode, for the light detection. For example, if the assay is carried out based on fluorescence, i.e. when the markers bonded to the analyte, do, when bonded to the analyte bonding agent and illuminated with light from the first light source, fluoresce (emit light due to fluorescence), the amount of fluorescence and thus the amount of light detected by the detecting unit, changes, e.g., increases (in case of a non-competitive assay), with an increasing amount of the analyte in the liquid. In case of a competitive assay, the amount of light detected by the detecting unit decreases with an increasing amount of the analyte in the liquid.

The first light source (and optional further light sources), such as the VCSEL(s), may or may not be comprised in the detecting unit, as will become clear from the below; and can be used in the color detection, in particular for the purpose of producing light to be detected. As will become clear in the following, the light to be detected can be (a) light emitted from the light source(s) - in which case a wavelength emitted from the light source(s) is identical with a wavelength detected; or (b) light emitted, e.g., from the markers, in reaction to illumination with the light emitted from the light source(s) - in which case a wavelength detected can differ from a wavelength emitted from the light source(s), as may be the case in photoluminescence-based assays, such as fluorescence-based assays.

In case of a fluorescence-based assay, the detecting unit can comprise an optical detector for detecting an intensity of the emitted fluorescent light, e.g., it can comprise a photodiode. The wavelengths detectable by the optical detector can in this case comprise a wavelength of the fluorescent light while not comprising any wavelength emitted by the first light source. The light source(s) in this case need not be comprised in the detecting unit.

In some embodiments, the first light source is a resonant-cavity light source, such as a VCSEL, and self-mixing interference (SMI) is used for the color detection. In this case, e.g., the first light source is structured and arranged
- to illuminate with light a test range in a test area of a carrier held in the carrier holder; and
- to couple back into the cavity of the first light source a portion of the light coming back from the test range.

Interaction, more particularly interference, of the light coupled back into the cavity with light being generated inside the cavity influences the light emitted from the light source. Thus the first light source is comprised in the detecting unit.

In this case, the detecting unit can comprise an optical detector for detecting an intensity of light emitted by the first light source, in particular wherein the measurement signal is derived from the optical detector. And/or the detecting unit comprises an electrical detector for detecting an electrical supply signal feeding the light source, in particular wherein the measurement signal is derived from the electrical detector.

SMI is a known interferometric measurement technique, usually used for distance measuring, However, it can also be used, as described, for color detection. Further details on this SMI-based color detection in assays, such as in LF testing, can be found in the European patent application No. 20 186 067.3 filed on July 15, 2020 by the same applicant - not yet published at the time of filing the instant patent application.

In some embodiments, the electrical consumers comprise a data transmission unit. The data transmission unit can in particular be provided for transmission of measurement signals, more particularly the measurement signals produced by the detecting unit. This makes possible to have the measurement signal analyzed externally from the monitoring device. E.g., the measurement signal can be transmitted to a cloud server, e.g., via a mobile communication device. An analysis of the measurement signal can then be carried out, e.g., by professional personnel, such as by health care professionals, and/or by specialized computer programs having access to the cloud server.

In some embodiments, the data transmission unit is, at least in part, identical to the energy receiving unit. It is possible to provide that identical parts, e.g., a coil, of the monitoring device, are used for both, receiving energy from the external device and transmitting the measurement signal. For example, this may be accomplished like in known technologies, such as in RFID technology and/or NFC technology.

In some embodiments, the measurement signal is transmitted to the external device. In other words, the external device on the one hand powers the monitoring device and on the other hand also receives the measurement signal.

For example, while the first light source (and optionally also the detecting unit) is powered by the external device, the measurement signal is transmitted to the external device.

In some embodiments, the external device (further) transmits the measurement signal or a signal, e.g., a data signal, derived therefrom; this may be accomplished, e.g., via a mobile communication standard.

In some embodiments, the monitoring device comprise a control unit, in particular, the electrical comsumers comprise the control unit. The control unit can be provided for controlling the first light source and, if present, further light sources and/or the detecting unit and/or the power supply unit and/or the data transmission unit.

In some embodiments, the monitoring device and the heterogeneous receptor-ligand assay device, respectively, is a hand-held device.

In some embodiments, the monitoring device and the heterogeneous receptor-ligand assay device, respectively, is a portable device.

In some embodiments, the monitoring device and the heterogeneous receptor-ligand assay device, respectively, is a disposable device.

In some embodiments, the monitoring device and the heterogeneous receptor-ligand assay device, respectively, is a single-use device.

The heterogeneous receptor-ligand assay device is a heterogeneous receptor-ligand assay device for detecting presence or amount of an analyte in a liquid, comprising a monitoring device as herein described, i.e. in particular a monitoring device according to the first aspect, or a monitoring device according to the second aspect, or a monitoring device according to both, the first aspect and the second aspect. It can furthermore comprise a carrier held in the carrier holder, the carrier comprising
- a sample pad for application of the liquid;
- a test area in which analyte bonding agents for bonding specifically to the analyte are fixed to the carrier;
- a control area in which marker bonding agents for bonding to markers are fixed to the carrier, said markers being markers for marking the analyte by bonding to the analyte;
wherein the carrier is structured such that the liquid is transported, in particular transported by capillary forces, from the sample pad to the test area and thereafter to the control area.

The control area is generally optional, thus can be dispensed with - in any herein-described embodiment.

In some embodiments, the assay is an immunoassay.

The heterogeneous receptor-ligand assay device can be, more specifically, a lateral flow test device. In particular, it can be is a lateral flow testing device for detecting presence or amount of an analyte in a liquid, which comprises a monitoring device as herein described, i.e. in particular a monitoring device according to the first aspect, or a monitoring device according to the second aspect, or a monitoring device according to both, the first aspect and the second aspect. It can furthermore comprise a carrier held in the carrier holder, and the carrier can furthermore comprise
- a sample pad for application of the liquid;
- a conjugate pad providing markers for marking the analyte by bonding to the analyte;
- a test area in which analyte bonding agents for bonding specifically to the analyte are fixed to the carrier;
- a control area in which marker bonding agents for bonding to the markers are fixed to the carrier;
wherein the carrier is structured such that the liquid is transported, in particular transported by capillary forces, from the sample pad to the conjugate pad, thereafter to the test area and thereafter to the control area. A mentioned above, the control area is generally optional.

The method according to the first aspect is a method for detecting presence or amount of an analyte in a liquid in a heterogeneous receptor-ligand assay, in particular in a lateral flow test, wherein the method comprises
- using a monitoring device comprising
   ∘ a power supply unit comprising an energy receiving unit, and
   ∘ one or more electrical consumers;
- receiving by means of the energy receiving unit energy from an external device;
- supplying the one or more electrical consumers with electrical energy originating from the energy received from an external device.

In particular, the receiving and the supplying can take place simultaneously.

The monitoring device can be, e.g., a monitoring device as herein described.

Optionally, one of the electrical consumers is a first light source, and the method can furthermore comprise
- illuminating a test range in a test area of a carrier with light from the first light source.

The carrier can in particular be a carrier as herein described.

Particularly, the steps of receiving, supplying and illuminating can take place simultaneously.

In some embodiments, one of the electrical consumers is a first light source, and the first light source is a VCSEL, in particular as proposed in the second aspect.

The method according to the second aspect is a method for detecting presence or amount of an analyte in a liquid in a heterogeneous receptor-ligand assay, e.g., by lateral flow testing, wherein the method comprises
- illuminating a test range in a test area of a carrier with light from a first light source which is a VCSEL.

The carrier can in particular be a carrier as herein described.

The method (according to any of the aspects) can furthermore comprise producing a measurement signal by means of a detecting unit, in particular wherein the detecting unit detects light emitted from the test range in reaction to illumination with light from the first light source.

In some embodiments, the detected light is luminescent (photoluminescent) light, such as fluorescent light, excited by the light from the first light source. In this case, a wavelength of the detected (e.g., fluorescent) light can differ from any wavelength emitted from the first light source or, more precisely, from any wavelength with which the liquid in the test range is illuminated by the first light source.

The luminescent light, e.g., fluorescent light, can in particular be emitted - in reaction to illumination with light from the first light source - from the markers bonded to the analyte when bonded to the analyte bonding agent, such as from marking agents of the markers.

In some embodiments, the carrier comprises
- a sample pad;
- a test area in which analyte bonding agents for bonding specifically to the analyte are fixed to the carrier;
- a control area in which marker bonding agents for bonding to markers are fixed to the carrier, said markers being markers for marking the analyte by bonding to the analyte, in particular by bonding specifically to the analyte;
and the method comprises
- applying the liquid to the sample pad;
- providing the markers;
- letting the markers bond to the analyte;
- letting the carrier transport the liquid from the sample pad to the test area and to the control area, in particular by capillary forces;
- letting the analyte bonding agents bond to the analyte at the test area;
- letting the marker bonding agents bond to the markers at the control area.

In particular, the carrier can comprise a conjugate pad providing the markers, and the method comprises letting the carrier transport the liquid from the sample pad to the conjugate pad, in particular by capillary forces, and the the letting the markers bond to the analyte takes place at the conjugate pad. Alternatively, the LFT can do without a conjugate pad, e.g., constituting a dip-stick-type LFT, and the letting the markers bond to the analyte comprises or is accomplished by dipping the carrier, into a substance comprising the markers, e.g., wherein the substance is a liquid such as a solution containig the markers.

The use according to the first aspect of the invention is the use of an energy receiving unit for receiving energy from an external device in a monitoring device for use in a heterogeneous receptor-ligand assay, e.g., in lateral flow testing, for detecting presence or amount of an analyte in a liquid. In particular, the monitoring device comprises one or more electrical consumers and a power supply unit for supplying said electrical consumers with electrical energy, and the energy receiving unit is comprised in the power supply unit.

A similar use according to the first aspect of the invention is the use of an energy receiving unit for receiving energy from an external device in a heterogeneous receptor-ligand assay device.

The use according to the second aspect of the invention is the use of a VCSEL as a light source (first light source) in a monitoring device for use in heterogeneous receptor-ligand assays, e.g., in lateral flow testing, for detecting presence or amount of an analyte in a liquid. In particular, the monitoring device can comprise a housing, the housing comprising a carrier holder for holding a carrier for transport of the liquid, and wherein the light source (first light source) is structured and arranged to illuminate with light a test range in a test area of a carrier held in the carrier holder.

A similar use according to the second aspect of the invention is the use of VCSEL as a light source (first light source) in a heterogeneous receptor-ligand assay device. In particular wherein the VCSEL used for color detection in the device.

As will be readily understood, features mentioned herein with respect to a method can analogously apply for a described device or use as well. And, similarly, features mentioned herein with respect to a device can analogously apply for a described method or use as well. And, similarly, features mentioned herein with respect to a use can analogously apply for a described method or device as well. The achievable effects correspond to each other.

Further embodiments and advantages emerge from the following description and the enclosed figures and from the dependent claims.

Below, the invention is described in more detail by means of examples and the included drawings. In the drawings, same reference numerals refer to same or analogous elements. The figures show:
- Fig. 1: a very schematic illustration of the method, including an LFT device;
- Fig. 2: a very schematic illustration of a side view of an LFT device;
- Fig. 3: a very schematic illustration of a side view of an LFT device.

The described embodiments are meant as examples or for clarifying the invention and shall not limit the invention.

Even though the figures and the following text relate to liquid flow testing (in particular as a non-competitive assay), this is merely an example, whereas the invention more generally relates to heterogeneous receptor-ligand assays.

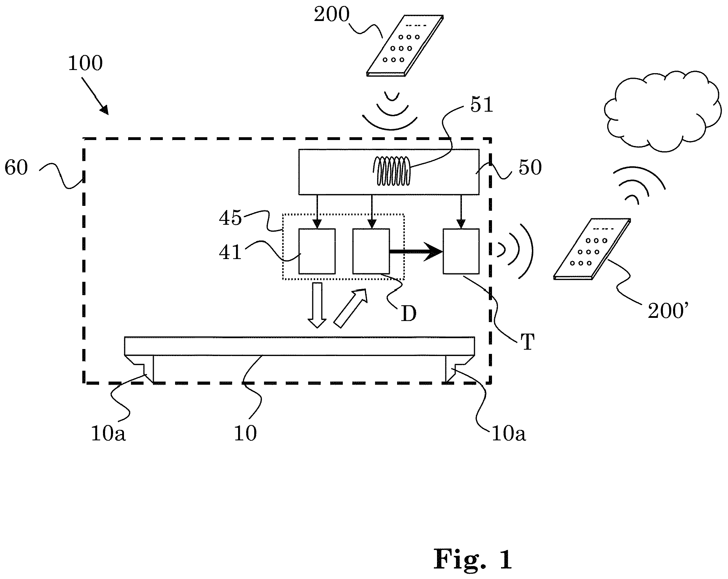

Fig. 1 shows a very schematic illustration of the method, including a heterogeneous receptor-ligand assay device 100, more particularly an LFT device 100. The LFT device 100 comprises a monitoring device and a carrier 10. The carrier 10 can be accomodated by the monitoring device. The monitoring device comprises a first light source 41 and a power supply unit 50. It may further comprise, as illustrated, a detecting unit D and an optional data transmission unit T. The LFT device 100 further comprises a housing 60 and a carrier holder 10a for holding the carrier 10.

Details regarding the LF testing are described further below.

The open arrows symbolize light. First light source 41, in particular embodied as a VSCEL, emits light towards a test range of the carrier 10. And detecting unit D detects light coming from the test range in reaction to the illumination with the light emitted by first light source 41. E.g., detecting unit D can comprise at least one photodetector. In some embodiments, the first light source comprises a cavity, and light is coupled back into the cavity, and detection is carried out based on SMI. In this case, first light source 41 can be a constituent of detecting unit D; and detection can take place optically, such as using a photodetector, or electrically, monitoring an electrical supply signal feeding the first light source 41. However, first light source 41 and detecting unit D can be separate parts, e.g., the detecting unit D comprising a photodetector, such as for detecting luminescent, e.g., fluorescent light emitted (from the test range) in reaction to illumination with light from first light source 41.

The electrical consumers (41; D; T) of the of the LFT device 100, and of the monitoring device, respectively, are supplied with electrical energy by power supply unit 50 which comprises an energy receiving unit 51, e.g., comprising a coil for inductive power transmission, as symbolized in Fig. 1. The thin arrows symbolize power supply connections.

This makes possible to operate the LFT device 100 and the monitoring device, respectively, without a battery: The power required for operation can be received, via the energy receiving unit 51, from an external device 200, such as from a smart phone.

This can be accomplished in a wireless fashion, e.g., based on RF waves, e.g., according to an RFID or NFC standard. Or it can be accomplished in a wirebound fashion, e.g., the energy receiving unit 51 comprising a connector, such as a socket, e.g., according to a USB standard.

The power supply unit 50 can furthermore comprise an AC-DC converter, in particular for converting an AC signal produced by the energy receiving unit, e.g., from inductive reception of electromagnetic waves, into a DC signal used for powering the consumers.

Detecting unit D outputs a measurement signal, illustrated by the thick arrow in Fig. 1, which is associated with the light coming from the test range.

Optionally, data transmission unit T is provided. It can receive the measurement signal from the detecting unit and transmit it to an external device 200', which however can be identical to external device 200. From there, the measurement signal or a signal derived therefrom can be further transmitted, e.g., to a cloud server, as symbolized by a cloud in Fig. 1.

During operation, each of the light emission from first light source 41, the detection by detecting unit D, and optionally also the data transmission by data transmission unit T can take place simultaneously with the receiving of energy from external device 200 - which, via energy receiving unit 51 and power supply unit 50, makes possible to power the electrical consumers.

The monitoring device can (optionally; not illustrated) also comprise a control unit controlling the operation of, e.g., one or more of the first light source 41, the detecting unit D, the data transmission unit T, the power supply unit 50.

The monitoring device can be considered to comprise a unit 45 for color detection, comprising the first light source 41 and the detecting unit D, and optionally also the control unit. In particular, by means of the unit 45 for color detection, the presence and/or intensity of a color can be determined.

The first light source 41 can in particular be a VCSEL. A VCSEL can be of very tiny size, and it can have a very low power consumption.

According to the second aspect of the invention, the first light source is a VCSEL, and the power supply unit 50 is generally optional, as is the detecting unit D and the data transmission unit T.

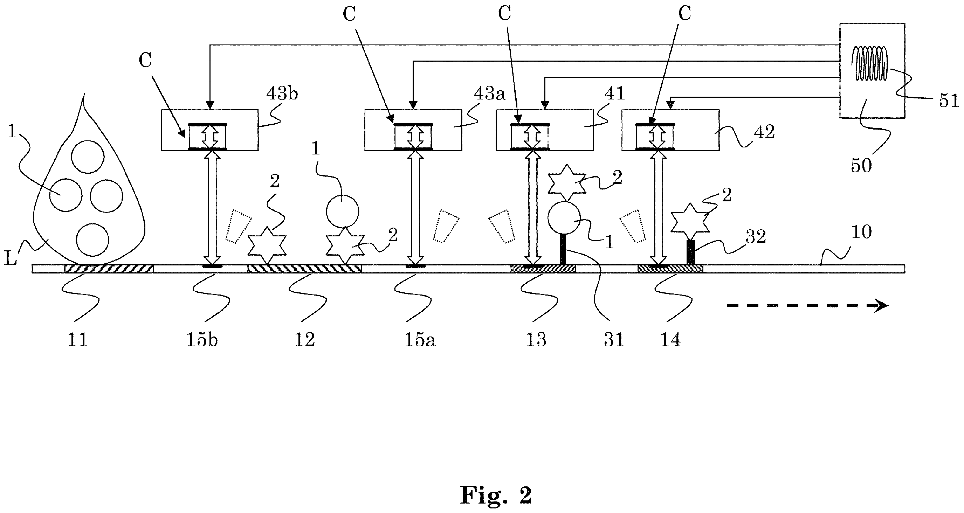

Fig. 2 shows a schematic illustration of a side view of an LFT device. This figure emphasizes the LFT aspect and the related method, and therefore some other details, such as a housing of the device are not illustrated in Fig. 2.

A carrier 10 accomodated in the device, e.g., held by carrier holder 10 (cf. Fig. 1), comprises a sample pad 11, a conjugate pad 12, a test area 13 and an optional control area 14. It further comprises two reference areas 15a, 15b, which are generally optional, and wherein, e.g., a single reference area can, in instances, be sufficient.

In particular, the carrier 10 can, in practice, comprise two or more constituents, e.g., pads, which are laminated one onto the other. For example (not shown): Carrier 10 can comprise a sample pad, and a first end portion of a conjugate part is laminated to a first end portion of the sample pad; and a detection pad of the carrier comprising the test area and the control area is laminated at its first end portion to a second end portion of the conjugate pad (opposite the first end portion of the conjugate pad); and a wicking pad of the carrier may be laminated to a second end portion of the detection pad (opposite the first end portion of the detection pad).

A liquid L representing a sample is applied to the sample pad 11. From there, it is transported by the carrier 10, e.g., by capillary forces, along a transport direction illustrated in the figures by the thick dashed arrow. The liquid L can (and in the illustrated case does) contain an analyte 1, such as a virus or an antigen.

On the conjugate pad 12, markers 2 are present which can bond to the analyte 1. Such a marker 2 can be, e.g., a conjugate comprising a ligand for bonding specifically to the analyte 1 and, bonded thereto, a marking agent such as a fluorescent agent, e.g., a fluorescent molecule, or a coloring agent, e.g., a gold particle or a latex particle.

The particles formed this way (marker 2 bonding to agent 1), when reaching the test area, can bond to analyte bonding agents 31 which are fixed to the carrier 10 in the test area 13. Accordingly, a concentration of the particles can increase there with time. The particles bonded to the analyte bonding agents 31 show a specific color (first color), e.g., such that an intensity of the color can increase with time and with concentration of the bonded particles in the test area 13. This can be detected, e.g., by detecting absorption, in particular at a wavelength of an absorption band of the bonded particles. This can be accomplished, e.g., based on SMI, the first light source 41 in this case constituting a part of the the detecting unit D. In another example, if the markers are fluorescent markers, this can be detected, e.g., by detecting the fluorescent light excited by the light emitted from first light source 41 and more particularly, by detecting an intensity of the fluorescent light.

Markers 2 not bonded to the analyte 1 are further transported by carrier 10 to the control area 14 and can bond to marker bonding members 32 which are fixed to the carrier 10 in the control area 14. The markers 2 bonded to the marker bonding agents 32 also show a specific color, such that, e.g., an intensity of that color can increase with time and concentration of these bonded particles in the control area 14.

The detection of the presence and/or the concentration of the respective bonded particles in the control area 14 can again be accomplished, e.g., using SMI. Due to the color, an absorption can be detected, wherein the absorption depends on the presence and/or the concentration of the respective bonded particles. Again, e.g., absorption or fluorescent light can be detected, as described above.

Therefore, the device comprises one or more light sources; in the illustrated example, four light sources 41, 42, 43a, 43b are shown. They all can be resonant-cavity light sources comprising a cavity C. In particular, they can all be VCSELs. Each cavity C has two end mirrors, one of which is the outcoupling mirror from which the respective light source emits light. Light produced in the light source circulates in the cavity, resonating therein. The hollow arrows in the figures symbolize light.

Light emitted from the respective light source illuminates a respective range (test range; control range; reference ranges) of the carrier 10 in the respective area 13, 14, 15a and 15b, respectively. These ranges are symbolized by short thick lines in Fig. 2.

In case of SMI-based detection, as illustrated in Fig. 2, a portion of the light is reflected back into the cavity C of the respective light source. Some of the light is, however, absorbed, in particular by the respective bonded particles in the test area 13 and in the control area 14.

Light re-entering the cavity C can influence the light generation in the light source, in particular due to interference with light present in the cavity, i.e. with light being produced in the light source. A notable coherence length of the light can therefore be advantageous. Generally, for example, lasers, e.g.,VCSELs and edge-emitting lasers (EELs) can be used. But also resonant-cavity light-emitting diodes (RC-LEDs) can be used.

This influence on the light generation can be detected, e.g., from monitoring a supply signal applied to the respective light source. In an example, the current drawn by a light source at a constant supply voltage can be a measurement signal - which is related to the influences to the light generation in the light source and thus to the absorption.

In case of fluorescent markers, the light emitted from the light sources excites fluorescence in the markers which is detected by the detecting unit which in Fig. 2 is symbolized by dotted trapezoids. E.g., each dotted trapezoid can represent a photodiode.

As illustrated in Fig. 2, each of the light sources is connected for power supply purposes to power supply unit 50 comprising an energy receiving unit 51.

The reference measurenment signals from the reference areas 15a, 15b (or one of them) can be used for calibration purposes, e.g., for monitoring the effect of a wetting of the carrier 10 by the liquid L. This can lead to more precise and/or more reliable results.

But also when the liquid has left the carrier (or at least the respective regions 13, 14, 15a, 15b), the reference measurenment signals from the reference areas 15a, 15b (or from one of them) can be used for calibration purposes, namely in order to compare absorption at an area (15a; 15b) without the color-bearing particles bonded to the carrier 10 to absorption at an area (13; 14) where the color-bearing particles are bonded to the carrier 10 (at least in case of presence of analyte 1 and/or markers 2).

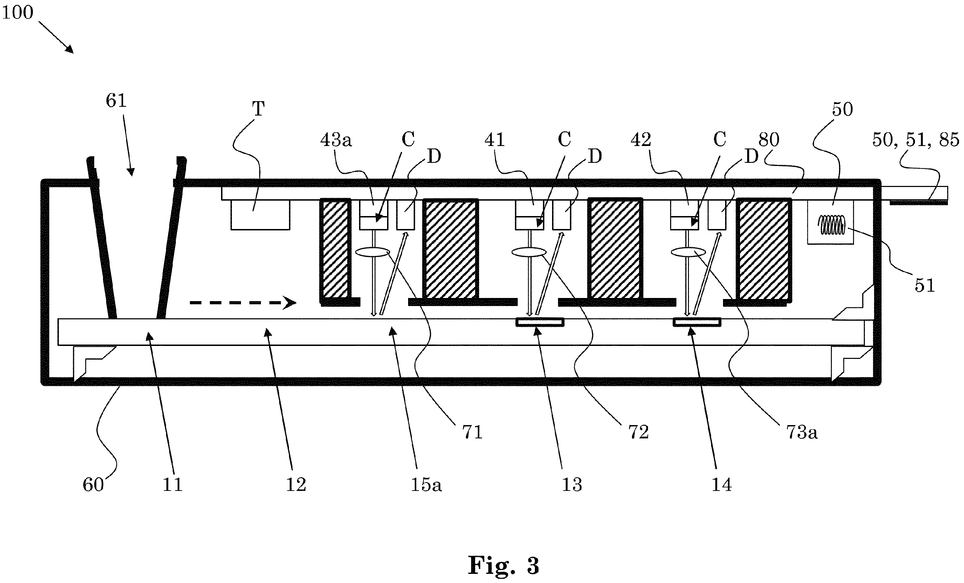

Fig. 3 shows a schematic illustration of a side view of an LFT device implementing, e.g., fluorescence-based detection. Many details have been described already in conjunction with Fig. 2. Accordingly, it is referred to above for that.

For the embodiments of both, Fig. 2 and Fig. 3, applies that any light source in excess of first light source 41 is merely an option.

The monitoring device comprises a housing 60 and may have a sample inlet 61. The housing 60 can comprise several baffles (thick lines or hatched in Fig. 3) to keep ambient light out of the housing and in particular away from the ranges (test range in test area 13, control range in control area 14, reference range in reference area 15a). Also the sample inlet 61 can serve as such a baffle.

The carrier 10 is held by a carrier holder (symbolized by the large angles in Fig. 3).

Optionally, for each light source, a lens 71, 72, 73a can be provided, disposed in the light path between the respective light source and the carrier 10. This way, the light beam exiting the respective light source can be, e.g., widened and/or parallelized. The lenses can optionally be attached to the respective light sources.

Furthermore, according to another option which however is not illustrated in Fig. 3, for each of the detectors of detecting unit D, a lens can be provided, in the light path between the respective range and the respective detector.

The LFT device 100 can further comprise a printed circuit board 80 on which the light sources and the detectors are mounted.

The device can also comprise a connector 85 which can be implemented, e.g., at the printed circuit board 80. The connector 85 can, as illustrated, comprise a portion of the printed circuit board 80. Power can be supplied to the LFT device via this connector 85. Connector 85 can be a constituent of the energy receiving unit 51 of power supply unit 50.

Alternatively to the connector 85 or in addition thereto, the LFT device 100 can comprise a receiving unit 51 for wireless energy transmission, e.g., comprising an induction coil. In case both are provided, they can be implemented in a single unit and/or share one or more common parts.

A data transmission unit T may be provided and may also be mounted on printed circuit board 80. It may be separate from (as illustrated) or - at least in part - identical to the energy receiving unit 51 (unlike shown in Fig. 3). E.g., data transmission unit T and energy receiving unit 51 may share one or more common parts, e.g., a coil, in particular an induction coil, serving both purposes: wireless reception of energy and wireless transmission of measurement signals.

## Claims

1. A monitoring device for use in a heterogeneous receptor-ligand assay for detecting presence or amount of an analyte in a liquid, the monitoring device comprising
- one or more electrical consumers;
- a power supply unit for supplying the one or more electrical consumers with electrical energy;
the power supply unit comprising an energy receiving unit for receiving energy from an external device; in particular wherein the heterogeneous receptor-ligand assay is a flow test, more particularly a lateral flow test.

2. The monitoring device according to claim 1, said energy receiving unit comprising a coil for wireless power transmission by electromagnetic induction.

3. The monitoring device according to claim 1 or claim 2, said power supply unit comprising an AC-DC converter for conversion of alternating current signals outputted by the energy receiving unit into direct current signals supplied to said one or more electrical consumers.

4. The monitoring device according to one of claims 1 to 3, the one or more electrical consumers comprising a first light source, in particular a first light source for emitting infrared light.

5. The monitoring device according to claim 4, wherein said first light source comprises a vertical-cavity surface-emitting laser.

6. The monitoring device according to one of claims 4 to 5, comprising
- a housing, the housing comprising a carrier holder for holding a carrier for transport of the liquid;
wherein the first light source is structured and arranged to illuminate with light a test range in a test area of a carrier held in the carrier holder.

7. The monitoring device according to one of claims 1 to 6, the one or more electrical consumers comprising a detecting unit comprising a detector, in particular a detector for detecting light, and in particular wherein the detecting unit produces a measurement signal associated with the presence or amount of the analyte in the liquid.

8. The monitoring device according to one of claims 1 to 7, the one or more electrical consumers comprising a data transmission unit, in particular for transmission of measurement signals.

9. A heterogeneous receptor-ligand assay device for detecting presence or amount of an analyte in a liquid, comprising a monitoring device according to one of claims 1 to 8 and a carrier held in the carrier holder, the carrier comprising
- a sample pad for application of the liquid;
- a test area in which analyte bonding agents for bonding specifically to the analyte are fixed to the carrier;
- a control area in which marker bonding agents for bonding to markers are fixed to the carrier, said markers being markers for marking the analyte by bonding to the analyte;
wherein the carrier is structured such that the liquid is transported, in particular transported by capillary forces, from the sample pad to the test area and thereafter to the control area;
in particular wherein the heterogeneous receptor-ligand assay device is a lateral flow test device, more particularly wherein the carrier comprises
- a conjugate pad providing the markers; and
the carrier is structured such that the liquid is transported, in particular transported by capillary forces, from the sample pad to the conjugate pad, thereafter to the test area and thereafter to the control area.

10. A method for detecting presence or amount of an analyte in a liquid in a heterogeneous receptor-ligand assay, in particular in a lateral flow test, wherein the method comprises
- using a monitoring device comprising
∘ a power supply unit comprising an energy receiving unit, and
∘ one or more electrical consumers, in particular the one or more electrical consumers comprising a first light source, more particularly a vertical-cavity surface-emitting laser;
- receiving by means of the energy receiving unit energy from an external device;
- supplying the one or more electrical consumers with electrical energy originating from the energy received from an external device;
in particular wherein the receiving and the supplying take place simultaneously.

11. The method according to claim 10, the one or more electrical consumers comprising a first light source, the method further comprising
- illuminating a test range in a test area of a carrier transporting the liquid with light from the first light source;
in particular wherein the receiving, the supplying and the illuminating take place simultaneously.

12. The method according to claim 11, wherein the first light source is a vertical-cavity surface-emitting laser, in particular a vertical-cavity surface-emitting laser for emission of infrared light.

13. The method according to claim 11 or claim 12, comprising carrying out, by means of a detecting unit of the monitoring device, the steps of
- detecting light emitted from the test area in reaction to the illumination with the light from the first light source, in particular wherein said emitted light is produced by luminescence, such as by fluorescence; and
- producing a measurement signal associated with the detected light.

14. The method according to one of claims 10 to 13, comprising using a carrier which comprises
- a sample pad;
- a test area in which analyte bonding agents for bonding specifically to the analyte are fixed to the carrier;
- a control area in which marker bonding agents for bonding to markers are fixed to the carrier, said markers being markers for marking the analyte by bonding to the analyte, in particular by bonding specifically to the analyte;
the method comprising
- applying the liquid to the sample pad;
- providing the markers;
- letting the markers bond to the analyte;
- letting the carrier transport the liquid from the sample pad to the test area and to the control area, in particular by capillary forces;
- letting the analyte bonding agents bond to the analyte at the test area;
- letting the marker bonding agents bond to the markers at the control area;
in particular wherein the carrier comprises a conjugate pad providing the markers and the method comprises letting the carrier transport the liquid from the sample pad to the conjugate pad, in particular by capillary forces, and the wherein the letting the markers bond to the analyte takes place at the conjugate pad.

15. The method according to one of claims 10 to 14, wherein the external device is a mobile communication device and/or a mobile computing device, in particular a smart phone or a mobile computer, such as a tablet computer.
